# EUROPEAN PATENT APPLICATION

(11) **EP 4 473 981 A1**
(43) Date of publication of application: **11.12.2024**
(21) Application number: 23787997.8
(22) Date of filing: 01.02.2023
(51) Int. Cl.: A61L 2/18, C12M 3/00, C12M 1/12

(54) **CULTURING DEVICE AND METHOD FOR STERILIZING CULTURING DEVICE**

(30) Priority: 15.04.2022 JP 2022067630
(71) Applicant: PHC Holdings Corporation, Tokyo 100-8403 (JP)
(72) Inventor: YOSHIDA, Kaori, Toon-Shi, Ehime 791-0395 (JP); OHTA, Akihiro, Toon-Shi, Ehime 791-0395 (JP); TEMMAN, Haruka, Toon-Shi, Ehime 791-0395 (JP); HITOMI, Tsugumasa, Toon-Shi, Ehime 791-0395 (JP)
(74) Representative: Grünecker Patent- und Rechtsanwälte PartG mbB
(86) International application number: PCT/JP2023/003199
(87) International publication number: WO 2023/199574

(57) **Abstract**

This culturing device has: a casing having a culturing chamber; a water vapor supply device having a liquid-receiving part for receiving water, a heater for heating the liquid-receiving part and generating water vapor, and a water vapor supply pipe for guiding the water vapor to the culturing chamber; a liquid supply device having liquid piping configured so as to enable a water storage part or a decontamination liquid storage part to be selectively attached thereto, and a liquid feed pump, the liquid supply device supplying, with the driving of the liquid feed pump, water stored in the water storage part or a decontamination liquid stored in the decontamination liquid storage part to the liquid-receiving part; and a control device. The control device controls the heater and the liquid feed pump so as to perform: a cooling process for channeling the water in the water storage part through the liquid piping and the liquid-receiving part and discharging the water from the water vapor supply pipe; and a decontamination process of channeling, after the cooling process, the decontamination liquid in the decontamination liquid storage part through the liquid piping and the liquid-receiving part and discharging the decontamination liquid from the water vapor supply pipe.

## Description

### Technical Field

The present disclosure relates to a culturing apparatus and a method for decontaminating a culturing apparatus.

### Background Art

A culturing apparatus (incubator) for culturing a culture such as a cell or a microorganism is known in the related art (see, for example, Patent Literature (hereinafter, referred to as PTL) 1).

A drawer is provided below a culture chamber in a culturing apparatus described in PTL 1. In the drawer, a heater, a tube pump, and a water storage are provided as a configuration for humidifying the culture chamber. Water stored in the water storage is dropped onto the heater by the tube pump at predetermined time intervals. Water dropped onto the heater is heated by the heater and turns into vapor. This vapor is guided into the culture chamber via the pipe portion, so that the culture chamber is humidified.

### Citation List

### Patent Literature

PTL 1
Japanese Patent Application Laid-Open No. 2021-78437

### Summary of Invention

### Technical Problem

Meanwhile, in a configuration, such as that of the culturing apparatus described in PTL 1, in which the culture chamber is humidified by using vapor, a tube and a pipe portion of the tube pump are contaminated.

An object of the present disclosure is to provide a culturing apparatus and a method for decontaminating a culturing apparatus that are capable of appropriately decontaminating a flow path of vapor and water for vapor generation.

### Solution to Problem

A culturing apparatus according to the present disclosure includes:
a housing including a culture chamber;
a vapor supply device including a liquid receiver that receives water, a heater that heats the liquid receiver to generate vapor, and a vapor supply pipe that guides the vapor to the culture chamber;
a liquid supply device including a liquid feeding pump and a liquid pipe that is configured to be selectively attachable to a water storage or a decontamination liquid storage, the liquid supply device supplying water stored in the water storage or a decontamination liquid stored in the decontamination liquid storage to the liquid receiver in accordance with driving of the liquid feeding pump; and
a control device,
wherein the control device controls the heater and the liquid feeding pump to perform
   a cooling process in which the water in the water storage is caused to pass through the liquid pipe and the liquid receiver and discharged from the vapor supply pipe without heating the liquid receiver, and
   after the cooling process, a decontamination process in which the decontamination liquid in the decontamination liquid storage is caused to pass through the liquid pipe and the liquid receiver and discharged from the vapor supply pipe.

In a method for decontaminating a culturing apparatus according to the present disclosure,
the culturing apparatus includes
   a housing including a culture chamber,
   a vapor supply device including a liquid receiver that receives water, a heater that heats the liquid receiver to generate vapor, and a vapor supply pipe that guides the vapor to the culture chamber, and
   a liquid supply device including a liquid feeding pump and a liquid pipe that is configured to be selectively attachable to a water storage or a decontamination liquid storage, the liquid supply device supplying water stored in the water storage or a decontamination liquid stored in the decontamination liquid storage to the liquid receiver in accordance with driving of the liquid feeding pump; and
the culturing apparatus performs
   a cooling process in which the water in the water storage is caused to pass through the liquid pipe and the liquid receiver and discharged from the vapor supply pipe without heating the liquid receiver to cool the liquid receiver and the vapor supply pipe, and
   after the cooling process, a decontamination process in which the decontamination liquid in the decontamination liquid storage is caused to pass through the liquid pipe and the liquid receiver and discharged from the vapor supply pipe to decontaminate the liquid pipe, the liquid receiver, and the vapor supply pipe.

### Advantageous Effects of Invention

According to the culturing apparatus and the method for decontaminating a culturing apparatus of the present disclosure, it is possible to appropriately decontaminate the flow path of vapor and water for vapor generation.

### Brief Description of Drawings

FIG. 1 is a schematic longitudinal section when an inside of a culturing apparatus is viewed from a right side;
FIG. 2 is a perspective view of the culturing apparatus;
FIG. 3 is a schematic diagram illustrating a connection state between a water tank, a liquid supply device, and a vapor supply device;
FIG. 4 is a schematic diagram illustrating a connection state between a dispenser, the liquid supply device, and the vapor supply device;
FIG. 5 is a flowchart of decontamination operation;
FIG. 6A is a diagram for explaining a cooling process in the decontamination operation;
FIG. 6B is a diagram for explaining a decontamination process in the decontamination operation; and
FIG. 6C is a diagram for explaining a replacement process in the decontamination operation.

### Description of Embodiments

### [Embodiment]

Hereinafter, embodiments of the present disclosure will be described.

### <Configuration of culturing apparatus>

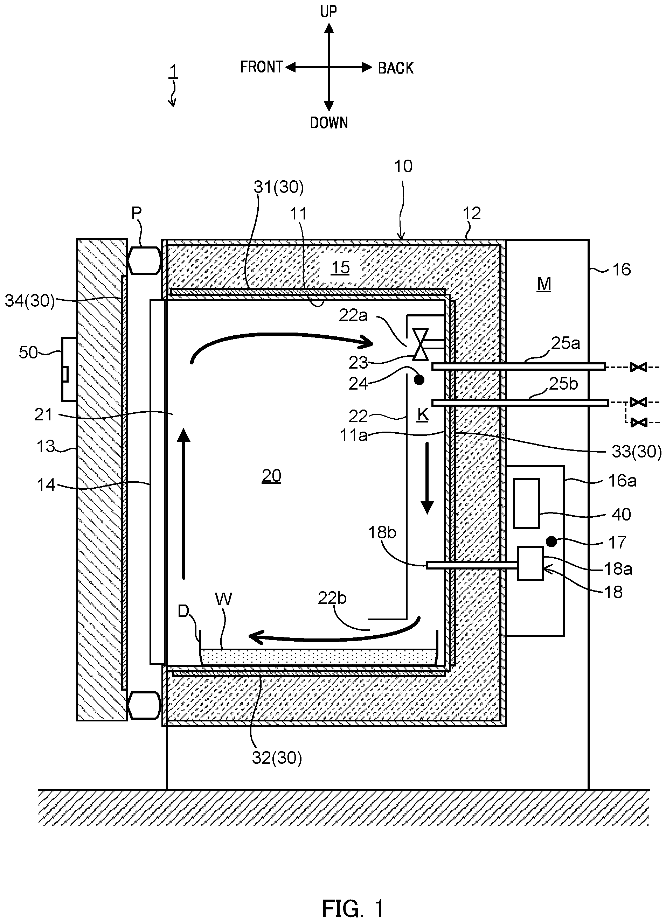

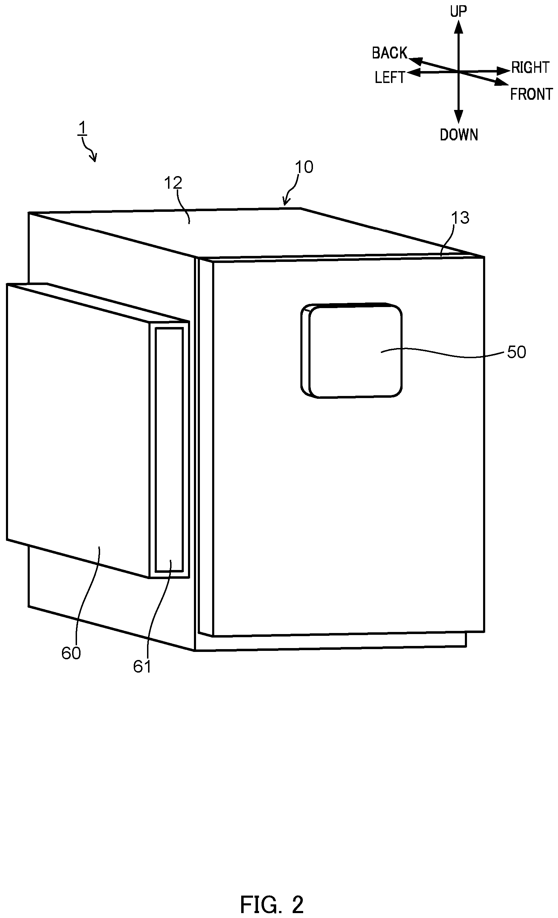

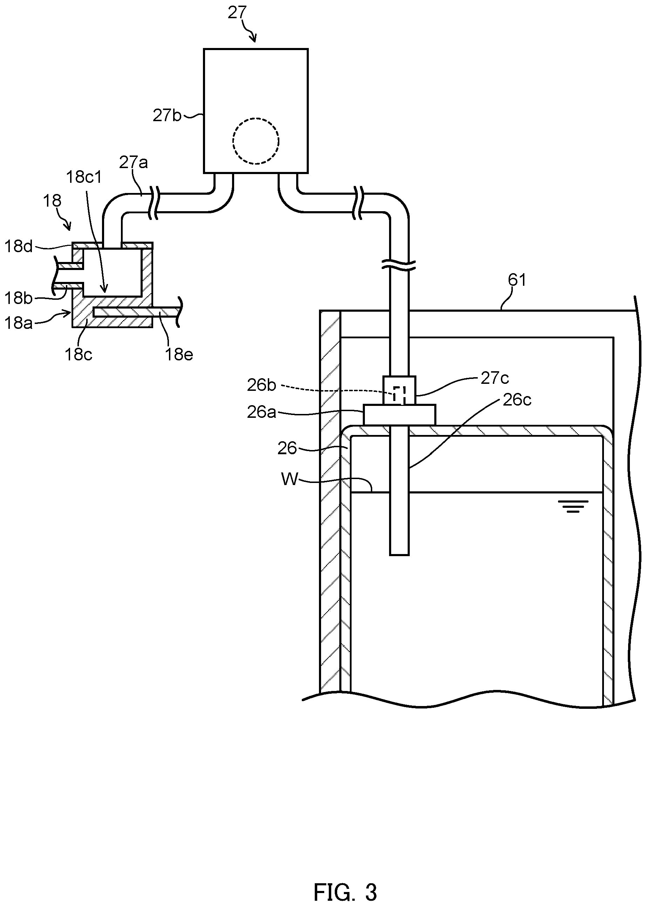

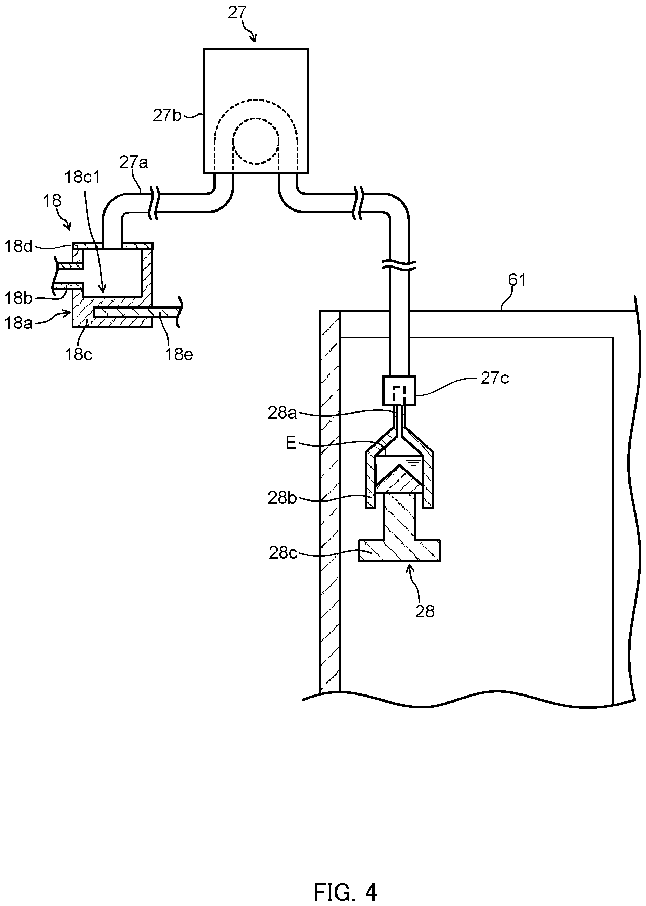

First, a configuration of a culturing apparatus will be described. FIG. 1 is a schematic longitudinal section when an inside of the culturing apparatus is viewed from a right side. FIG. 2 is a perspective view of the culturing apparatus. FIG. 3 is a schematic diagram illustrating a connection state between a water tank, a liquid supply device, and a vapor supply device. FIG. 4 is a schematic diagram illustrating a connection state between a dispenser, the liquid supply device, and the vapor supply device. Hereinafter, a front side is a side in which a user of the culturing apparatus faces, and a back side is a side opposite to the front side. Further, the right side is a right side viewed from the user facing the culturing apparatus, and a left side is a side opposite to the right side. An upper side is an upper side in a case where the culturing apparatus is installed on a horizontal surface, and a lower side is a side opposite to the upper side.

First, a schematic configuration of the inside of culturing apparatus 1 will be described based on FIG. 1. Culturing apparatus 1 includes box-shaped housing 10. Culture chamber 20 for culturing a culture such as a cell or a microorganism is formed inside housing 10. Culture chamber 20 is configured in such a way that at least one shelf (not illustrated) can be detachably installed therein at a predetermined position in the vertical direction. Housing 10 includes inner box 11, outer box 12, outer door 13, and inner door 14.

Inner box 11 is formed in a box shape including culture chamber 20 therein. Opening 21 of culture chamber 20 is formed on the front surface of inner box 11. Outer box 12 is formed in a box shape. Outer box 12 is disposed to cover portions of inner box 11 except for opening 21. Heat insulating material 15 is disposed between inner box 11 and outer box 12.

Outer door 13 and inner door 14 open and close opening 21. Packing Pis disposed at an outer edge of outer door 13. Operator 50 is disposed on an outer surface of outer door 13. Operator 50 receives instructions to start, stop, and decontaminate culturing apparatus 1, as well as inputs of various set values of culture chamber 20. The various set values of culture chamber 20 are a set temperature, set humidity, a set concentration of O₂ gas, a set concentration of CO₂ gas, and the like. Operator 50 includes a display that displays a state of culturing apparatus 1.

Heater 30 that heats culture chamber 20 is disposed in housing 10. Heater 30 includes top-surface heater 31 disposed on the upper side of inner box 11, bottom-surface heater 32 disposed on the lower side of inner box 11, rear-surface heater 33 disposed on the back side of inner box 11, side-surface heaters (not illustrated) disposed on the left and right sides of inner box 11, and outer door heater 34 disposed on outer door 13.

Duct 22 is disposed in culture chamber 20. Duct 22 is formed on the back surface of inner box 11 to vertically extend. Gas passage K is formed inside duct 22. In gas passage K, circulation fan 23 is disposed. By operating circulation fan 23, air in culture chamber 20 is sucked in from suction port 22a formed in an upper portion of duct 22, and this air is blown out to culture chamber 20 from blow-out port 22b provided in a lower portion of duct 22. As a result, forced circulation of the air as indicated with bold arrows is performed.

In duct 22, room temperature sensor 24 and gas supply devices 25a and 25b are disposed. Room temperature sensor 24 detects a temperature of culture chamber 20. Room temperature sensor 24 is disposed in a vicinity of suction port 22a and detects the temperature of air sucked in from suction port 22a. Gas supply devices 25a and 25b supply, to culture chamber 20, adjustment gas (for example, CO₂ gas, O₂ gas, and N₂ (nitrogen) gas) that adjust the O₂ gas concentration and the CO₂ gas concentration in culture chamber 20.

Humidification pan D for storing a liquid (specifically, water) for humidification is installed between the lower portion of duct 22 and the bottom surface of inner box 11. Water stored in humidification pan D is decontaminated by ultraviolet irradiation performed by a UV-LED (not illustrated). A humidity sensor (not illustrated) that detects humidity in culture chamber 20 is disposed outside duct 22.

The rear surface and the bottom surface of outer box 12 in housing 10 are covered with cover 16. A space between the rear surface of outer box 12 and cover 16 forms mechanical room M for disposing various types of equipment. Mechanical room M is provided with electrical box 16a. Electrical box 16a stores control device 40.

In addition, culturing apparatus 1 further includes outside air temperature sensor 17 and vapor supply device 18. Outside air temperature sensor 17 detects an ambient temperature of culturing apparatus 1. Vapor supply device 18 supplies vapor to culture chamber 20. Vapor supply device 18 includes vapor generator 18a and vapor supply pipe 18b.

Vapor generator 18a is disposed in electrical box 16a. Vapor supply pipe 18b supplies the vapor generated by vapor generator 18a to culture chamber 20.

Next, a configuration of culturing apparatus 1 visible from the outside will be described based on FIG. 2. Tank storage 60 is provided on the left side surface of outer box 12 of housing 10. Tank storage 60 is configured in such a way that drawer 61 can be pulled out and put in in a front-back direction.

Next, a specific configuration for performing humidification of culture chamber 20 or decontamination of vapor and water for vapor generation will be described based on FIGS. 3 and 4. FIGS. 3 and 4 are diagrams illustrating connection states in each configuration, and are not diagrams accurately illustrating a positional relationship in each configuration in the up-down, front-back, and right-left directions. As illustrated in FIG. 3, for humidification of culture chamber 20, water tank 26, vapor supply device 18, liquid supply device 27, and humidification pan D are used.

Water tank 26 is an example of a water storage of the present disclosure that stores water W. Water tank 26 is stored in drawer 61 while fixed into drawer 61.

Vapor generator 18a of vapor supply device 18 includes liquid receiver 18c, lid 18d, and heater 18e.

Liquid receiver 18c is formed in a block shape from a material having heat conductivity. Liquid receiver 18c includes recess 18c1 recessed downward. Vapor supply pipe 18b is inserted into a front-side portion of liquid receiver 18c. Vapor supply pipe 18b is inserted such that the inside of vapor supply pipe 18b communicates with the inside of recess 18c1 and the opening exposed to recess 18c1 is located above the bottom surface of recess 18c1. Lid 18d closes the upper surface of liquid receiver 18c.

Heater 18e is disposed in a vicinity of recess 18c1 in liquid receiver 18c. Heater 18e is electrically connected to control device 40. Heater 18e generates vapor by heating water supplied to recess 18c1 of liquid receiver 18c to evaporate the water, based on control by control device 40. This vapor is guided into culture chamber 20 via vapor supply pipe 18b, so that culture chamber 20 is humidified.

Liquid supply device 27 includes liquid tube 27a and tube pump 27b.

Liquid tube 27a is a liquid pipe of the present disclosure, and is made of resin or rubber. A part of liquid tube 27a is located in tube pump 27b. A portion of one end side of liquid tube 27a is attached to lid 18d of liquid receiver 18c such that the inside of liquid tube 27a and the inside of recess 18c1 of liquid receiver 18c communicate with each other. As illustrated in FIG. 3, tube connection port 27c for guiding water W in water tank 26 to liquid receiver 18c is provided at the other end of liquid tube 27a. Cap connection port 26b is provided in cap 26a of water tank 26. An upper side portion of cap connection port 26b is configured to be connected to tube connection port 27c. Tank tube 26c is attached to a lower side portion of cap connection port 26b. A part of tank tube 26c is located in water W.

Further, as illustrated in FIG. 4, tube connection port 27c of liquid tube 27a is configured so that ejector 28a of dispenser 28 can be inserted therein in order to guide ethanol E in dispenser 28 to liquid receiver 18c. That is, liquid tube 27a is configured to be selectively attachable to water tank 26 or dispenser 28.

In the embodiment of the present disclosure, tube connection port 27c is configured of a male-shaped luer lock standard connector, and cap connection port 26b and ejector 28a of the dispenser are configured of female-shaped luer lock standard connectors. As described above, liquid tube 27a is configured to be selectively attachable to water tank 26 or dispenser 28 by using connectors with the same standard for tube connection port 27c, cap connection port 26b, and ejector 28a, and by setting female shapes of cap connection port 26b and ejector 28a to the same shape. The shapes of tube connection port 27c, cap connection port 26b, and ejector 28a may be reversed in terms of male and female. In this case, there is an advantage in that connection to a general dispenser having a luer shape such as a general syringe can be performed.

Tube pump 27b is an example of a liquid feeding pump of the present disclosure. Tube pump 27b is electrically connected to control device 40. Tube pump 27b rotates a rotor (not illustrated) that squeezes a part of liquid tube 27a to supply water W in water tank 26 or ethanol E in dispenser 28 to liquid receiver 18c, based on the control by control device 40. Tube pump 27b is configured to adjust an amount of water W per unit time, which is supplied to liquid receiver 18c by increasing or decreasing a rotation speed of the rotor.

Dispenser 28 is an example of a decontamination liquid storage of the present disclosure. Ethanol E stored in dispenser 28 is an example of a decontamination liquid of the present disclosure. Dispenser 28 is formed in a size such that the dispenser can be put in drawer 61 in place of water tank 26. Dispenser 28 includes syringe 28b that includes ejector 28a, and piston 28c that reciprocates in syringe 28b. A scale (not illustrated) indicating an amount of ethanol E in syringe 28b is displayed on syringe 28b. As described above, by using dispenser 28 in which the scale is displayed, the user can fill dispenser 28 with ethanol E with a minimum usage amount. In addition, when a usage amount of ethanol E is too large, there is a concern that an ethanol vapor concentration in culture chamber 20 exceeds an explosion concentration in a case where all of used ethanol E is volatilized, and thus, it is also desirable that a capacity of dispenser 28 is 10 ml or less in order to also suppress the use of excessive ethanol E due to a user's operation mistake. Further, in tube pump 27b, when ethanol E in dispenser 28 is supplied into liquid receiver 18c, liquid feeding of ethanol E is performed while piston 28c is retracted. That is, when the capacity of dispenser 28 is large and piston 28c is large, there is a concern that piston 28c is less likely to be retracted and the liquid feeding of ethanol E is delayed. Accordingly, it is desirable that the capacity of dispenser 28 is 20 ml or less.

### <Operation of culturing apparatus>

Next, operations of culturing apparatus 1 will be described.

### <Culturing operation>

First, a culturing operation of culturing a culture will be described as the operation of culturing apparatus 1. Before starting the culturing operation, the user installs humidification pan D in culture chamber 20 and places, for example, a petri dish in which the culture is put in, on a shelf. When information on starting the culturing operation is input into operator 50 by the user, control device 40 controls circulation fan 23, gas supply devices 25a and 25b, and heater 30 such that the concentrations and temperatures of the gases become suitable for culturing the culture in housing 10 (culture chamber 20). When bottom-surface heater 32 generates heat, water stored in humidification pan D evaporates, and culture chamber 20 is humidified so that the humidity in culture chamber 20 reaches the target value.

Control device 40 selects one of normal humidification control and rapid humidification control based on the information input into operator 50 by the user during the culturing operation, and humidifies culture chamber 20 such that the humidity detected by the humidity sensor reaches a target value. The normal humidification control is control in which culture chamber 20 is humidified by evaporating the water in humidification pan D with heat generation of bottom-surface heater 32 without operating vapor supply device 18. The rapid humidification control is control in which culture chamber 20 is humidified by evaporating the water in humidification pan D with the heat generation of bottom-surface heater 32 and by operating vapor supply device 18, and therefore, culture chamber 20 can be humidified quickly as compared with the normal humidification control.

In the rapid humidification control, control device 40 drives heater 18e of vapor supply device 18 to heat liquid receiver 18c, and drives tube pump 27b of liquid supply device 27 to supply water W in water tank 26 to liquid receiver 18c. At this time, control device 40 intermittently drives tube pump 27b such that water W is dropped into liquid receiver 18c at a predetermined interval. Vapor is generated from liquid receiver 18c into which water W is dropped, and the vapor is guided to culture chamber 20, thereby humidifying culture chamber 20.

When the user inputs information to end the culture operation into operator 50, the control device stops circulation fan 23, gas supply devices 25a and 25b, and heater 30, and in a case where vapor supply device 18 is operated, control device 40 stops vapor supply device 18.

### <Decontamination operation>

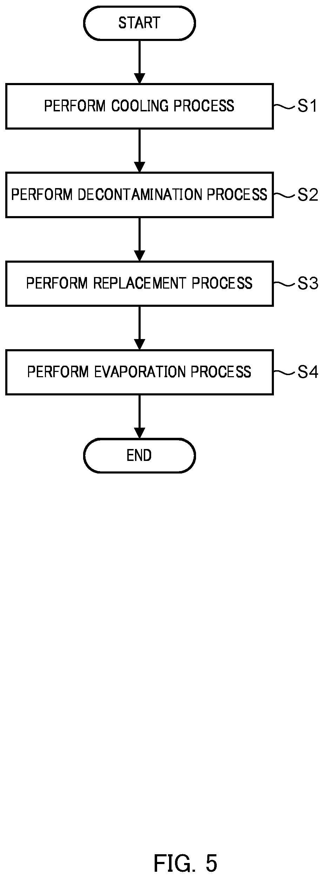

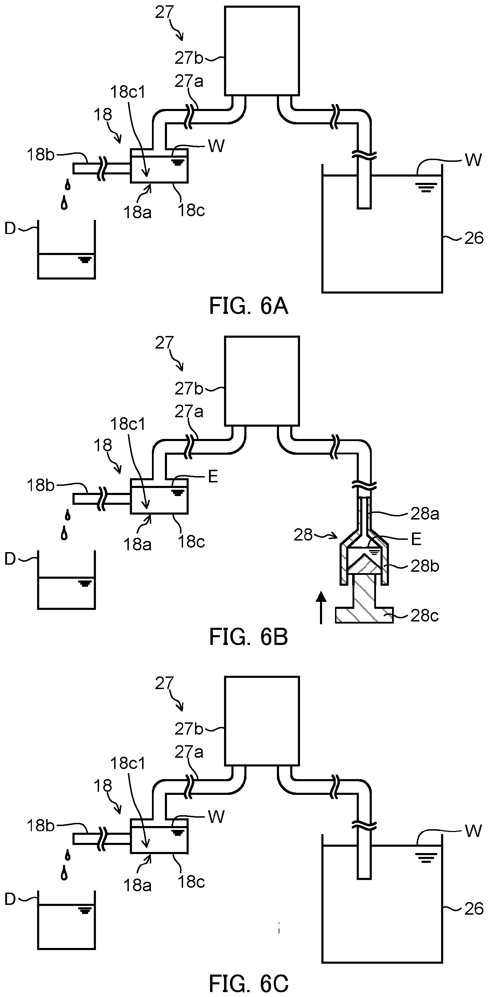

Next, as the operation of culturing apparatus 1, a decontamination operation will be described with reference to FIGS. 5, 6A, 6B, and 6C. The decontamination operation is performed for decontaminating liquid tube 27a, liquid receiver 18c, and vapor supply pipe 18b, that is, for decontaminating the flow path of vapor and water for vapor generation. FIG. 5 is a flowchart of the decontamination operation. FIG. 6A is a diagram for explaining a cooling process in the decontamination operation. FIG. 6B is a diagram for explaining a decontamination process in the decontamination operation. FIG. 6C is a diagram for explaining a replacement process in the decontamination operation.

When the user inputs information to start the decontamination operation into operator 50 while the culture operation is not being performed, control device 40 causes a setup instruction to be displayed in operator 50. The setup instruction includes, for example, an instruction to dispose humidification pan D below vapor supply pipe 18b and an instruction for confirming a water amount in water tank 26. In the decontamination operation, water W and ethanol E are discharged to humidification pan D, and thus, it is preferable for the user to empty humidification pan D based on the setup instruction. Thereafter, the user performs a work based on the setup instruction and then presses a setup completion button displayed in operator 50. In the setup instruction, an instruction to evacuate the culture in culture chamber 20 (not illustrated) may be further displayed. As a result, it is possible to prevent the user from forgetting to evacuate the culture and to prevent unintended culture annihilation.

When control device 40 detects an operation of the setup completion button, control device 40 starts the cooling process as illustrated in FIG. 5 (step S 1). In the cooling process, control device 40 drives tube pump 27b to supply water W in water tank 26 to liquid receiver 18c. At this time, control device 40 controls tube pump 27b such that water W is continuously supplied to liquid receiver 18c. That is, control device 40 controls tube pump 27b such that a supply amount of water W per unit time to liquid receiver 18c increases to be larger than that in a case where the rapid humidification control is performed. By controlling the water amount in this manner, it is possible to decrease a temperature of liquid receiver 18c quickly.

As illustrated in FIG. 6A, water W supplied to liquid receiver 18c is accumulated in recess 18c1, and is eventually discharged to humidification pan D via vapor supply pipe 18b. Control device 40 may cause operator 50 to display the temperature of liquid receiver 18c during the cooling process. Control device 40 controls, after a predetermined time has elapsed since the start of the cooling process, tube pump 27b such that supply of water W to liquid receiver 18c ends, and thus, ends the cooling process. Control device 40 may end the cooling process based on an operation of operator 50 by the user, or may end the cooling process when the control device detects that the temperatures of liquid receiver 18c and vapor supply pipe 18b have become equal to or lower than a first predetermined temperature. The above-described first predetermined temperature is desirably 100°C or lower, at which supplied water W no longer boils at liquid receiver 18c. By such a cooling process, liquid receiver 18c and vapor supply pipe 18b whose temperatures have been increased by the culturing operation are cooled. In the embodiment of the present disclosure, vapor supply device 18 includes an evaporator temperature sensor (not illustrated) that measures the temperature of liquid receiver 18c, and water W is supplied to liquid receiver 18c until the temperature of liquid receiver 18c reaches 60°C or lower. That is, the supply of water W to liquid receiver 18c is ended after it is confirmed that the temperature of liquid receiver 18c has decreased sufficiently. As a result, even when a situation occurs in which cooling becomes insufficient due to a shortage of water W in the water tank 26, a next process does not proceed, and thus, it is possible to perform the decontamination operation safely without exposing ethanol E to a high temperature.

Next, control device 40 causes operator 50 to display a first liquid exchange instruction to exchange water tank 26 for dispenser 28. When the user exchanges water tank 26 for dispenser 28, the user pulls out drawer 61 to the front, then pulls tube connection port 27c of liquid tube 27a out from cap connection port 26b of water tank 26, and takes water tank 26 out from drawer 61. Subsequently, the user inserts ejector 28a of dispenser 28 filled with a set amount of ethanol E into tube connection port 27c of liquid tube 27a. When the user releases a hand from dispenser 28, dispenser 28 is no longer fixed within drawer 61 but is left hanging from liquid tube 27a. The user pushes drawer 61 to be put in tank storage 60 together with dispenser 28. After performing a work based on the first liquid exchange instruction, the user presses a first liquid exchange completion button displayed in operator 50.

When control device 40 detects an operation of the first liquid exchange completion button, control device 40 starts the decontamination process as illustrated in FIG. 5 (step S2). In the decontamination process, control device 40 drives tube pump 27b to supply ethanol E in dispenser 28 to liquid receiver 18c. At this time, control device 40 controls tube pump 27b such that ethanol E is continuously supplied to liquid receiver 18c in the same manner as in the case of the cooling process.

As illustrated in FIG. 6B, ethanol E supplied to liquid receiver 18c is accumulated in recess 18c1, and is eventually discharged to humidification pan D via vapor supply pipe 18b. When ethanol E in dispenser 28 runs out, control device 40 stops tube pump 27b to end the decontamination process. Control device 40 may end the decontamination process after a predetermined time has elapsed since the start of the decontamination process with ethanol E still remaining in dispenser 28, or may end the decontamination process based on the operation of operator 50 by the user. By such a decontamination process, liquid tube 27a, liquid receiver 18c, and vapor supply pipe 18b are decontaminated with ethanol E.

Next, control device 40 causes a second liquid exchange instruction to exchange dispenser 28 for water tank 26 to be displayed in operator 50. After the user pulls out drawer 61 to the front, the user pulls ejector 28a of dispenser 28 out from tube connection port 27c of liquid tube 27a, and stores water tank 26, in which water W is stored, in drawer 61. Then, the user inserts cap connection port 26b of water tank 26 into tube connection port 27c of liquid tube 27a and pushes drawer 61 to be put in tank storage 60. After performing a work based on the second liquid exchange instruction, the user presses a second liquid exchange completion button displayed in operator 50.

When control device 40 detects an operation of the second liquid exchange completion button, control device 40 starts the replacement process as illustrated in FIG. 5 (step S3). In the replacement process, control device 40 controls tube pump 27b such that water W is continuously supplied to liquid receiver 18c in the same manner as in the case of the cooling process.

As illustrated in FIG. 6C, water W supplied to liquid receiver 18c is accumulated in recess 18c1, and is eventually discharged to humidification pan D via vapor supply pipe 18b. Control device 40, after a predetermined time has elapsed from the start of the replacement process, controls tube pump 27b to end the supply of water W to liquid receiver 18c, thereby ending the replacement process. Control device 40 may end the replacement process based on the operation of operator 50 by the user. By such a replacement process, ethanol E that has adhered to liquid tube 27a, liquid receiver 18c, and vapor supply pipe 18b is replaced with water W.

Next, control device 40 starts an evaporation process as illustrated in FIG. 5 (step S4). In the evaporation process, control device 40 controls heater 18e of vapor supply device 18 such that liquid receiver 18c is heated. Control device 40 stops heater 18e to end the evaporation process after a predetermined time has elapsed after the start of the evaporation process or when the temperature of liquid receiver 18c reaches a second predetermined temperature or higher. The above-described second predetermined temperature is desirably 100°C or higher, at which water W in liquid receiver 18c surely is evaporated. By such an evaporation process, water W in heated liquid receiver 18c is evaporated to be discharged into culture chamber 20 via vapor supply pipe 18b. Further, water W that has adhered to vapor supply pipe 18b is also evaporated and is discharged into culture chamber 20. In the embodiment of the present disclosure, the evaporation process in which the temperature of liquid receiver 18c is maintained at 100°C or higher for 1 minute or longer is continued. That is, the evaporation process is ended after confirming that water W in liquid receiver 18c has been surely evaporated sufficiently. As a result, it is possible to prevent water W that may include ethanol E remaining in liquid receiver 18c is evaporated from affecting the culture, when the culturing operation starts.

Next, control device 40 causes operator 50 to display a screen indicating the end of the decontamination operation. The user discards the liquid (water W and ethanol E) in humidification pan D and returns humidification pan D to culture chamber 20.

As described above, control device 40 performs the decontamination process using ethanol E after decreasing the temperature of liquid receiver 18c by the cooling process using water W. Therefore, even in a case where the decontamination operation is performed immediately after the culturing operation, the decontamination of the flow path of vapor and water for vapor generation can be safely performed by supplying ethanol E to liquid receiver 18c with a low temperature. Further, since the cooling process forcibly decreases the temperature of liquid receiver 18c, decontamination can be performed in a short time as compared with a case where the decontamination process is performed after leaving the liquid receiver until the temperature decreases. Thus, it is possible to appropriately perform decontamination of the flow path of vapor and water for vapor generation.

In addition, control device 40 performs, after the decontamination process, the replacement process of replacing ethanol E having adhered to liquid tube 27a, liquid receiver 18c, and vapor supply pipe 18b with water W. Therefore, it is possible to prevent the culturing operation from being performed while the ethanol E component remains in the flow path of vapor and water for vapor generation, and it is possible to culture the culture appropriately. Further, it is possible to start the culturing operation quickly without waiting for ethanol E to naturally volatilize.

In addition, after the replacement process, control device 40 performs the evaporation process of evaporating water W having adhered to liquid receiver 18c and vapor supply pipe 18b. Therefore, the culturing operation can start in a state in which water W used for the decontamination operation does not remain in liquid receiver 18c and vapor supply pipe 18b. Further, it is possible to start the culturing operation early without waiting for water W adhered to liquid receiver 18c and vapor supply pipe 18b to be naturally dried.

### [Variation of embodiment]

It is needless to say that the present disclosure is not limited to the embodiments described thus far, and that various modifications can be made without departing from the scope of the present disclosure. The above-described embodiments and variations described below may be combined in any manner in an applicable range.

For example, it is not necessary to perform the evaporation process, and in this case, it is preferable to wait until liquid receiver 18c and vapor supply pipe 18b are naturally dried and then start the culturing operation. Further, it is not necessary to perform the replacement process and the evaporation process, and in this case, it is preferable to wait until ethanol E having adhered to liquid tube 27a, liquid receiver 18c, and vapor supply pipe 18b naturally volatilizes, and then start the culturing operation.

The supply amount of water W per unit time to liquid receiver 18c when the replacement process is performed may be the same as that in a case of performing the rapid humidification control.

Although tube pump 27b has been shown as an example of the liquid feeding pump, a pump capable of feeding a liquid, such as a diaphragm type, a piston type, a plunger type, or the like may be applied. As the decontamination liquid storage, a configuration other than that of dispenser 28, such as a tank with the same shape as that of water tank 26, may be applied, or as the water storage, a configuration other than water tank 26, such as the dispenser, may be applied.

Although ethanol E has been shown as an example of the decontamination liquid, a liquid with a decontamination effect, such as isopropyl alcohol (IPA) may be applied.

This application is entitled to and claims the benefit of Japanese Patent Application No. 2022-67630, filed on April 15, 2022, the disclosure of which including the specification, claims, drawings and abstract is incorporated herein by reference in its entirety.

The present disclosure can be applied to a culturing apparatus and a method for decontaminating a culturing apparatus.

### Reference Signs List

1 Culturing apparatus
10 Housing
11 Inner box
12 Outer box
13 Outer door
14 Inner door
15 Heat insulating material
16 Cover
16a Electrical box
17 Outside air temperature sensor
18 Vapor supply device
18a Vapor generator
18b Vapor supply pipe
18c Liquid receiver
18c1 Recess
18d Lid
18e Heater
20 Culture chamber
21 Opening
22 Duct
22a Suction port
22b Blow-out port
23 Circulation fan
24 Room temperature sensor
25a, 25b Gas supply device
26 Water tank
26a Cap
26b Cap connection port
27 Liquid supply device
27a Liquid tube
27b Tube pump
27c Tube connection port
28 Dispenser
28a Ejector
28b Syringe
28c Piston
30 Heater
31 Top-surface heater
32 Bottom-surface heater
33 Rear-surface heater
34 Outer door heater
40 Control device
50 Operator
60 Tank storage
61 Drawer
D Humidification pan
E Ethanol
K Gas passage
M Mechanical room
P Packing
W Water

## Claims

1. A culturing apparatus comprising:
a housing including a culture chamber;
a vapor supply device including a liquid receiver that receives water, a heater that heats the liquid receiver to generate vapor, and a vapor supply pipe that guides the vapor to the culture chamber;
a liquid supply device including a liquid feeding pump and a liquid pipe that is configured to be selectively attachable to a water storage or a decontamination liquid storage, the liquid supply device supplying water stored in the water storage or a decontamination liquid stored in the decontamination liquid storage to the liquid receiver in accordance with driving of the liquid feeding pump; and
a control device,
wherein the control device controls the heater and the liquid feeding pump to perform
a cooling process in which the water in the water storage is caused to pass through the liquid pipe and the liquid receiver and discharged from the vapor supply pipe without heating the liquid receiver, and
after the cooling process, a decontamination process in which the decontamination liquid in the decontamination liquid storage is caused to pass through the liquid pipe and the liquid receiver and discharged from the vapor supply pipe.

2. The culturing apparatus according to claim 1, wherein
after the decontamination process, the control device controls the liquid feeding pump to perform a replacement process in which the water in the water storage is caused to pass through the liquid pipe and the liquid receiver and discharged from the vapor supply pipe.

3. The culturing apparatus according to claim 2, wherein
the control device controls the liquid feeding pump such that a water supply amount per unit time to the liquid receiver when the replacement process is performed is larger than a water supply amount per unit time when the culture chamber is humidified by the vapor.

4. The culturing apparatus according to claim 2 or 3, wherein
after the replacement process, the control device controls the heater to perform an evaporation process in which the liquid receiver is heated.

5. A method for decontaminating a culturing apparatus, wherein:
the culturing apparatus includes
a housing including a culture chamber,
a vapor supply device including a liquid receiver that receives water, a heater that heats the liquid receiver to generate vapor, and a vapor supply pipe that guides the vapor to the culture chamber, and
a liquid supply device including a liquid feeding pump and a liquid pipe that is configured to be selectively attachable to a water storage or a decontamination liquid storage, the liquid supply device supplying water stored in the water storage or a decontamination liquid stored in the decontamination liquid storage to the liquid receiver in accordance with driving of the liquid feeding pump; and
the culturing apparatus performs
a cooling process in which the water in the water storage is caused to pass through the liquid pipe and the liquid receiver and discharged from the vapor supply pipe without heating the liquid receiver to cool the liquid receiver and the vapor supply pipe, and
after the cooling process, a decontamination process in which the decontamination liquid in the decontamination liquid storage is caused to pass through the liquid pipe and the liquid receiver and discharged from the vapor supply pipe to decontaminate the liquid pipe, the liquid receiver, and the vapor supply pipe.

6. The method according to claim 5, wherein
the culturing apparatus performs
after the decontamination process, a replacement process in which the water in the water storage is caused to pass through the liquid pipe and the liquid receiver and discharged from the vapor supply pipe to replace the decontamination liquid adhered to the liquid pipe, the liquid receiver, and the vapor supply pipe with the water.

7. The method according to claim 6, wherein
the culturing apparatus performs
after the replacement process, an evaporation process in which the liquid receiver is heated to evaporate the water adhered to the liquid receiver and the vapor supply pipe.
